# EUROPEAN PATENT APPLICATION

(11) **EP 1 347 062 A1**
(43) Date of publication of application: **24.09.2003**
(21) Application number: 01999674.3
(22) Date of filing: 10.12.2001
(51) Int. Cl.: C12Q 1/68

(54) **METHOD OF DETECTING ABNORMAL GENE**

(30) Priority: 08.12.2000 JP 2000003740
(71) Applicant: Kyushu TLO Company, Limited, Fukuoka-shi, Fukuoka 812-8581 (JP)
(72) Inventor: GOTO, Masahiro, Fukuoka-shi, Fukuoka 819-0044 (JP); PIAO, Lianchun, Fukuoka-shi, Fukuoka 813-0016 (JP); FURUSAKI, Shintaro, Fujisawa-shi, Kanagawa 251-0002 (JP)
(74) Representative: Robertson, James Alexander
(86) International application number: JP0110784
(87) International publication number: WO02046469

(57) **Abstract**

Disclosed is a new technology for use in gene diagnosis, which enables simple and highly sensitive detection of an abnormal gene. A nucleic acid oligomer having a base sequence of a normal gene of interest is hybridized in a reversed micelle with a probe having a base sequence complementary to said base sequence of the normal gene; the nucleic acid oligomer having the base sequence of the normal gene is hybridized in another reversed micelle with a probe having a base sequence complementary to a base sequence of an abnormal gene partly altered from the base sequence of the normal gene; and the rates of the hybridization reactions are measured to examine the difference therebetween. The rates of the hybridization reactions can be measured, for example, by measuring change in ultraviolet light absorbance.

## Description

### Technical Field

The present invention relates to a novel method for detecting abnormal genes.

### Background Art

As the Human Genome Project launched in the late 1980s is scheduled for completion early in the 21st century, the sequence of all human nucleotide bases, as many as three billion, will soon be determined, and all human genes, nearly one hundred thousand according to one view, be identified. The identification of a number of genes that cause hereditary disorders or are involved in a variety of biological functions in the human body can be expected to highlight the importance of gene diagnosis as a science for detecting gene abnormalities and predicting, preventing and treating certain diseases.

The method most often employed in gene diagnosis comprises hybridizing a normal gene and an abnormal gene with the corresponding probes (oligonucleotides), and analyzing the results by gel electrophoresis to detect the abnormal gene. However, this method indispensably requires labeling of the probes with an radioactive isotope, a fluorescent dye, an enzyme or the like, making the method disadvantageous from the perspective of safety, cost, automation and so on. The hybridization reactions are carried out in an aqueous media in which the rates of the reactions depend upon the DNA concentrations in the aqueous solution and other parameters. In the conventional method, however, the hybridization reactions are not conducted under a reaction environment in which the reaction rates are controlled so that the reactions proceed in an optimal manner. Thus, the conventional method is disadvantageous in that it is restricted with respect to the gene samples and probes employed and also with respect to the detection sensitivity achieved thereby.

The object of the present invention is to provide a new technology that can be used in gene diagnosis to detect abnormal genes in a simple and convenient manner with high sensitivity.

### Disclosure of the Invention

Through extensive studies, the present inventors found that the above-mentioned object can be achieved by hybridizing a target gene and a probe for the gene incorporated in a reversed micelle used as the reaction field for the hybridization and monitoring the hybridization reaction rate in the reversed micelle.

Thus, according to the present invention, there is provided a method for detecting an abnormal gene, which comprises hybridizing, in a reversed micelle, a nucleic acid oligomer having a base sequence of a normal gene of interest with a probe having a base sequence complementary to said base sequence of the normal gene; hybridizing, in another reversed micelle, the nucleic acid oligomer having the base sequence of the normal gene with a probe having a base sequence complementary to a base sequence of an abnormal gene partly altered from the base sequence of the normal gene; and measuring the rates of the hybridization reactions to examine the difference therebetween.

In a preferred embodiment of the present invention the hybridization reactions are carried out, in a single reversed micelle into which there have been injected the nucleic acid oligomer having the base sequence of the normal gene and the probe having the base sequence complementary to the base sequence of the normal gene, and in another single reversed micelle into which there have been injected the nucleic acid oligomer having the base sequence of the normal gene and the probe having the base sequence complementary to the base sequence of the abnormal gene.

In a preferred embodiment of the present invention, the measurement of the rates of the hybridization reactions is carried out by measuring change in ultraviolet light absorbance, particularly absorbance at 260nm.

### Brief Description of the Drawings

Figure 1 shows the results of time-course absorbance measurements conducted to study the effect of temperature on hybridization in an aqueous solution.
Figure 2 shows the results of ultraviolet absorption spectra measurements conducted to study the effect of cation concentration on hybridization in an aqueous solution.
Figure 3 shows the measured ultraviolet absorption spectra of an oligonucleotide derived from human tumor suppressor gene p53 in a reversed micelle.
Figure 4 shows an example of time-course absorbance during hybridization reactions between fully-matched oligonucleotides and between mismatched oligonucleotides in reversed micelles, measured for detecting an abnormal gene in accordance with the present invention.
Figure 5 shows an example of time-course absorbance during hybridization reactions between fully-matched oligonucleotides and between mismatched oligonucleotides in reversed micelles, measured for detecting an abnormal gene in accordance with the present invention.
Figure 6 shows the correlation between initial rate of hybridization in a reversed micelle and the location of the altered site, calculated for detecting an abnormal gene in accordance with the present invention.

### Best Mode for Carrying Out the Invention

In accordance with the method of the present invention, the interior of a reversed micelle, an aggregate of nanomolecules, is used as a reaction field for a nucleic acid hybridization reaction in which the rate of the reaction is measured to detect an abnormal gene. Such kinetic detection of a specific gene is a completely novel process not available hitherto. The novel detection process is based on the following principles:
(1) The greater is the degree of complementariness between base sequences, the higher is the rate of hybridization of the nucleic acids in forming a hybrid. When mismatched bases are present, therefore, the rate of hybridization and the thermal stability of the hybrid decrease in proportion to the number and locations of mismatches. Thus, the present invention makes it possible to evaluate the number and locations (sites) of mismatches existing in an abnormal gene by comparing the hybridization rates for the abnormal gene and for the normal gene in reversed micelles, thereby enabling abnormal gene detection.
(2) Since the present invention uses the interior of a reversed micelle as the reaction field for the nucleic acid hybridization, the rate of the hybridization can be optimally controlled by changing the diameter (size) of the reversed micelle, the number of the reversed micelles, the formation mode of the reversed micelles and other parameters.
(3) A nucleic acid can be concentrated in a reversed micelle. Thus, even with a nucleic acid having such low concentration that it does not form a hybrid in a conventional aqueous medium, it is possible to conduct the hybridization in the reversed micelle, thereby enabling the detection of an abnormal gene with a high sensitivity.

Based upon the above-mentioned principles, the method of the present invention is practiced as follows: A nucleic acid oligomer (oligonucleotide) having a base sequence (a nucleotide sequence) of a normal gene of interest is hybridized, in a reversed micelle, with a probe having a base sequence complementary to the base sequence of the normal gene. Further, (another sample of) the nucleic acid oligomer (oligonucleotide) having the base sequence of the normal gene is hybridized, in another reversed micelle, with a probe having a base sequence complimentary to a base sequence of an abnormal gene partly altered from the base sequence of the normal gene. The abnormal gene can be detected by measuring the rates of these hybridization reactions to examine the difference therebetween.

As well known, the term "reversed micelle" means an aggregate of molecules formed in an organic medium through high self-organization, more specifically, an aggregate of surface active molecules in an organic solvent in the approximate shape of a sphere in which the hydrophilic portions (hydrophilic functional or atomic groups) are in the center and hydrophobic portions are on the outside (on the side of the organic solvent).

Such hybridization in a reversed micelle can be practiced in a number of modes. For example, a reversed micelle is prepared into which is injected a nucleic acid oligomer having a base sequence of a normal gene of interest, while a reversed micelle is prepared into which is injected a probe having a base sequence complementary to the sequence of the normal gene. Then the two micelles are admixed to form a micelle, in the interior of which the hybridization between the oligomer and the probe proceeds. Similarly, a reversed micelle is prepared into which is injected (another sample of) the nucleic acid oligomer having the base sequence of the normal gene, while a reversed micelle is prepared into which is injected a probe having a base sequence complementary to a base sequence of an abnormal gene partly altered from the base sequence of the normal gene. Then the two reversed micelles are admixed to form another reversed micelle. The rates of the hybridizations in these reversed micelles are measured to examine the difference between the rates of the hybridizations. While this method effectively detects whether there may be mismatch(es) due to the abnormal gene, it generally takes some time to detect the location(s) of the mismatch(es) (cf. Example 1 below).

Thus, according to a preferred embodiment of the present invention, a nucleic acid oligomer having a base sequence of a normal gene of interest is injected into a reversed micelle. Into the "same" reversed micelle is also injected a probe having a base sequence complementary to the base sequence of the normal gene. Similarly, (another sample of) the nucleic acid oligomer having the base sequence of the normal gene of interest is injected into another reversed micelle. Into the "same" other reversed micelle is also injected a probe having a base sequence complementary to the base sequence of the abnormal gene. Then the hybridization reactions are carried out in these reversed micelles to measure the rates of the reactions and examine the difference therebetween. In this method, the rates of the hybridization reactions are enhanced because the oligonucleotides are brought into direct contact with each other. Thus, the method makes it possible to detect not only the presence of possible mismatch(es) but also the location(s) of such mismatch(es) (cf. Example 2 below).

The incorporation of a nucleic acid oligomer of a normal gene or a probe therefor into a reversed micelle is carried out in the form an aqueous solution prepared by dissolving such oligonucleotide in a buffer having a pH and an ionic strength similar to the physiological conditions.

As well known, a probe having a base sequence complementary the base sequence of an abnormal gene of interest can be obtained by subjecting the double-stranded nucleic acid to be inspected under high alkaline conditions or heating such nucleic acid to form a single-stranded nucleic acid.

While the nucleic acid analyzed by the present invention is generally DNA, the present invention can also be applied to the analysis of RNA such as mRNA. Thus, the term "hybridization" used with respect to the present invention refers not only a hybridization where there is formed a DNA-DNA hybrid, but also to DNA-RNA hybridization or RNA-RNA hybridization.

The surface active agent (surfactant) used in the present invention to prepare a reversed micelle is not restricted so far as it is capable of forming an aggregate of the molecules in an organic solvent due to self-organization as mentioned previously. While any of ionic (anionic, cationic or amphoteric) surface active agents and nonionic surface active agents can be used, an anionic surface active agent is preferably used. A nucleic acid such as DNA is anionic and therefore repulsive against the inner wall of a reversed micelle made of the anionic surface active agent. Since it therefore does not bind to the inner wall, the hybridization proceeds smoothly. Examples of surface active agents for use in preparing a reversed micelle for the present invention include, but are not limited to, sodium di-2-ethylhexyl sulfosuccinate (an anionic surface active agent known as AOT), dioleyl phosphate, sodium dodecyl sulfonate, and mixtures thereof. Examples of organic solvents for use as a media in preparing reversed micelle of an above-mentioned surface active agent include, but are not limited to, iso-octane, hexane, cyclohexane, dodecane, toluene, and chloroform.

In the practice of the present invention, such a surface active agent as mentioned above is generally used at a Wo (water/surface active agent ratio) in the range of 5 to 50. Wo is a parameter that controls the size (diameter) of a reversed micelle. The Wo value of the reversed micelle prepared for the hybridization is preferably greater in proportion as the length of the oligonucleotides to be hybridized is greater.

While for the detection of an abnormal gene the method of the present invention is generally suitable for application to the hybridization of oligonucleotides composed of 15 to 50 bases, it can also be applied to longer oligonucleotides (e.g. an oligonucleotide composed of 100 bases).

The rate of hybridization in a reversed micelle can be measured by any means capable of detecting the structural change of a nucleic acid due to the hybrid formation, including, for example, an UV apparatus (ultraviolet spectrophotometer), a CD apparatus (circular dichroism spectrophotometer), or a DSC apparatus (differential scanning calorimeter). It is preferred to measure the change in light absorbance in the ultraviolet region with an UV apparatus, because this enables easy detection of the structural change due to the hybridization through a simple operation. The measurement of change in light absorbance in the ultraviolet region is carried out, for example, by measuring the time-course change in light absorbance at 260nm so as to qualitatively detect the progress of the hybridization reaction. Alternatively, the rate of a hybridization reaction can be quantitatively evaluated by calculating the relative initial rate of the reaction from the slope of the plot of the absorbance against the time of the hybridization reaction.

### Examples

The present invention will now be more fully described with reference to the following examples, which are not for restricting the invention.

### Example 1: Preparatory Experiment

In order to determine the conditions for the measurement according to the method of the present invention for detecting an abnormal gene, a preparatory experiment was conducted in which hybridizations in aqueous solutions were studied.

### (1) DNA samples:

The following are synthetic oligonucleotides each having a sequence composed of 20 bases, for use in the experimental hybridizations between fully-matched DNAs and those between mismatched DNAs. ε denotes absorption coefficient at 260nm.
Normal gene: tumor-suppressor-gene p53
Nucleotide 1: 5'-GCTTTGAGGTGCGTGTTTGT-3' (SEQ ID NO:1) (ε 183100 Mcm⁻¹)
Probe having a base sequence complementary to tumor-suppressor-gene p53
Nucleotide 2: 5'-CGAAACTCCACGCACAAACA-3' (SEQ ID NO:2) (ε 197300 Mcm⁻¹)
Abnormal gene altered at position 13 of tumor-suppressor-gene p53
Nucleotide 3: 5'-CGAAACTCCACGAACAAACA-3' (SEQ ID NO:3) (ε 203100 Mcm⁻¹)
Nucleotide 4: 5'-CGAAACTCCACGTACAAACA-3' (SEQ ID NO:4) (ε 200600 Mcm⁻¹)
Abnormal gene altered at position 1 of tumor-suppressor-gene p53
Nucleotide 5: 5'-TGAAACTCCACGCACAAACA-3' (SEQ ID NO:5) (ε 198300 Mcm⁻¹)
Abnormal gene altered at position 7 of tumor-suppressor-gene p53
Nucleotide 6: 5'-CGAAACCCCACGCACAAACA-3' (SEQ ID NO:6 (ε 196400 Mcm⁻¹)

In order to learn the secondary structures of these oligonucleotides, calculations were made based on the respective sequences to determine the minimum stacking length and minimum free energy of the oligonucleotides. The results are as follows, from which it was confirmed that each of the oligonucleotides assumes a conventional single-stranded structure in an aqueous solution
Nucleotide 1: Minimum Stacking Length:2 ; Minimum Free Energy: 0.29 Kcal/mol
Nucleotide 2: Minimum Stacking Length:2 ; Minimum Free Energy: 1.59 Kcal/mol
Nucleotide 3: Minimum Stacking Length:2 ; Minimum Free Energy: 1.59 Kcal/mol
Nucleotide 4: Minimum Stacking Length:2 ; Minimum Free Energy: 1.59 Kcal/mol
Nucleotide 5: Minimum Stacking Length:2 ; Minimum Free Energy: 1.90 Kcal/mol
Nucleotide 6: Minimum Stacking Length:2 ; Minimum Free Energy: 1.59 Kcal/mol

### (2) Effect of temperature on rate of hybridization:

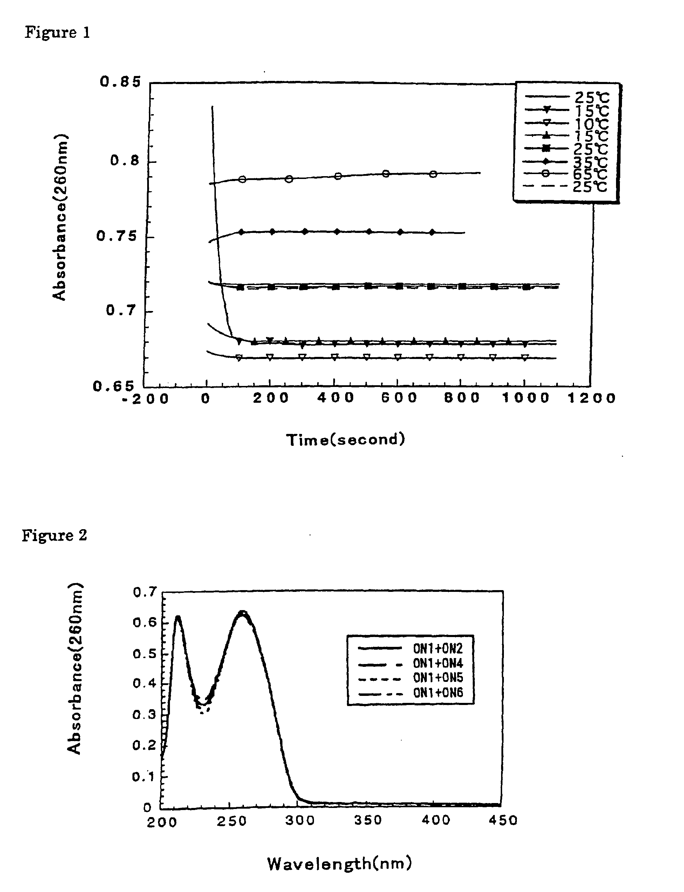

A DNA sample for fully-matched hybridization between complementary nucleotides was prepared. The sample was composed of 10mM of Tris, 1mM of EDTA (2Na), 100mM of NaCl, 2.017 µM of Nucleotide 1 and 2.057 µM of Nucleotide 2 and had a pH of 7.0. Time-course change in absorbance at 260nm was measured at different temperatures, i.e. 25, 15, 10, 15, 25, 35, 65, and 25°C. The results are shown in Figure 1.

As seen from Figure 1, absorbance at 260nm decreased with decreasing temperature. This indicates that hybridization proceeded more quickly as the temperature is lower. Based on this result and taking operational ease into consideration, it was decided to carry out the hybridization reaction at 15°C.

### (3) Effect of monovalent cation on rate of hybrid formation:

Measurements of absorption spectra in the region of 200nm to 800nm were made at 15°C using 1 ml each of the following Samples A, B, C and D.
Sample A: 10 mM of Tris, 1 mM of EDTA (2Na), 700 mM of NaCl, 1.75 µM of Nucleotide 1, 1.75 µM of Nucleotide 2, pH 7.0.
Sample B: 10 mM of Tris, 1 mM of EDTA (2Na), 700 mM of NaCl, 1.75 µM of Nucleotide 1, 1.75 µM of Nucleotide 4, pH 7.0.
Sample C: 10 mM of Tris, 1 mM of EDTA (2Na), 700 mM of NaCl, 1.75 µM of Nucleotide 1, 1.75 µM of Nucleotide 5, pH 7.0.
Sample D: 10 mM of Tris, 1 mM of EDTA (2Na), 700 mM of NaCl, 1.75 µM of Nucleotide 1, 1.75 µM of Nucleotide 6, pH 7.0.

The results are shown in Figure 2. As can be seen from Figure 2, in the presence of 700mM of sodium ion, a monovalent cation, the absorbance patterns of ON1 + ON2 (a fully-matched DNA), ON1 + ON4, ON1 + ON5, and ON1 + ON6 (mismatched DNAs) are quite similar to each other. It is also noted that the absorbance patterns show no substantial difference from those under the respective single-stranded conditions. The results thus suggest that hybridization reaction is quite slow under the above-mentioned conditions and also that the cation concentration has no substantial effect on the rate of the hybridization reaction.

Other parameters, such as the pH of the buffer (in the interior of reversed micelle) in which an oligonucleotide is dissolved, and the organic solvent in which a reversed micelle is formed, may influence the hybridization reaction. No particular consideration is given here to the influence of such pH and organic solvent since the method of the present invention is applied to gene diagnosis conducted under physiological conditions as a prerequisite.

### Example 2: Hybridization caused by fusion of reversed micelles

This illustrates an example of the detection of an abnormal gene through hybridization occurring in a reversed micelle formed by the fusion of two reversed micelles, wherein one reversed micelle injected with a small amount of an oligonucleotide and another reversed micelle injected with a small amount of another oligonucleotide whose sequence is fully-matched or mismatched to the first-mentioned oligonucleotide, are admixed to form the fused reversed micelle.

### (1) Formation of the preparatory reversed micelles:

To 3 ml of 50 mM AOT solution in iso-octane (an organic phase) was injected 50 µl of an aqueous solution of 198.0 µM Nucleotide 1 together with 10 mM Tris and 1 mM EDTA (2Na) having a pH of 7.0 (an aqueous phase) to form a reversed micelle as Sample A. In a similar manner there were prepared Samples B through E as summarized in Table 1.

**Table 1**

| | Organic Phase | Aqueous Phase | Apparent DNA Concentration Concentration |
|---|---|---|---|
| Sample A | Surfactant: AOT 50mM Solvent: Iso-octane | 10mM Tris, 1mM EDTA(2Na), pH 7.0, 198.0 µM Nucleotide 1 | 3,500µM |
| Sample B | Surfactant: AOT 50mM Solvent: Iso-octane | 10mM Tris, 1mM EDTA(2Na), pH 7.0, 198.0 µM Nucleotide 2 | 3,500 µM |
| Sample C | Surfactant: AOT 50mM Solvent: Iso-octane | 10mM Tris, 1mM EDTA(2Na), pH 7.0, 198.0 µM Nucleotide 4 | 3,500 µM |
| Sample D | Surfactant: AOT 50mM Solvent: Iso-octane | 10mM Tris, 1mM EDTA(2Na), pH 7.0, 198.0 µM Nucleotide 5 | 3,500 µM |
| Sample E | Surfactant: AOT 50mM Solvent: Iso-octane | 10mM Tris, 1mM EDTA(2Na), pH 7.0, EDTA(2Na), pH 7.0, 198.0 µM Nucleotide 6 | 3,500 µM |

### (2) Measurements:

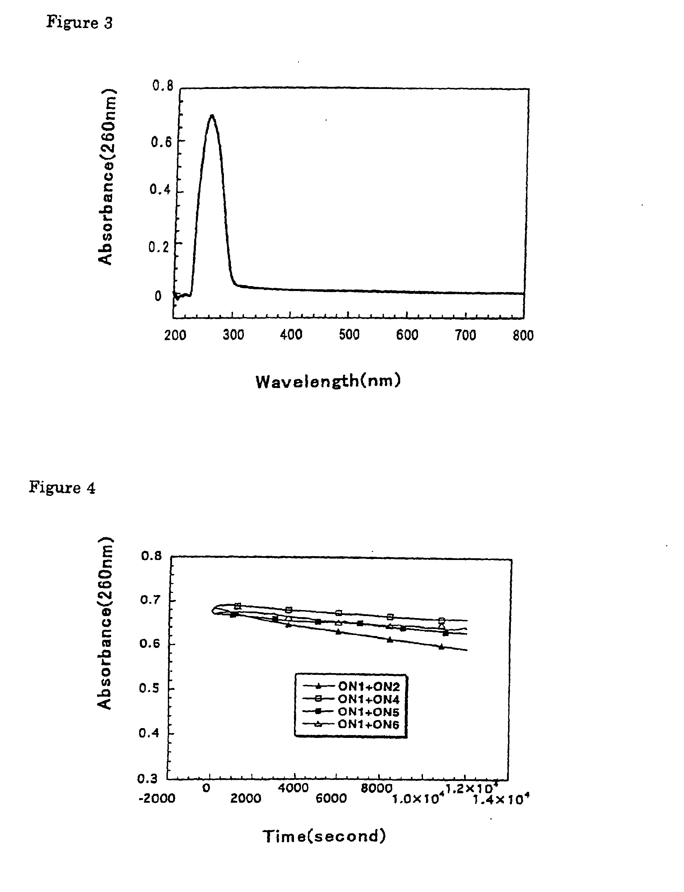

1 ml of Sample A was placed in an UV cell and measured for absorption spectra in the region of 200 nm to 800 nm at a temperature of 15°C. The results are shown in Figure 3. The absorption spectrum (absorbance) observed with the reversed micelle injected with Nucleotide 1 was not different from that with the aqueous solution of the nucleotide. It can thus be seen that the oligonucleotide was homogeneously introduced into the reversed micelle without being denatured.

0.5 ml of Sample A and 0.5 ml of Sample B were admixed so as to start a hybridization reaction (a fully-matched hybridization) at l5°C. Change in absorbance at 260 nm over the course of time was measured from immediately after the start of the reaction. In a similar manner, change in absorbance over the course of time during hybridization reaction at 15°C was measured for each for Sample A + Sample C; Sample A + Sample D; and Sample A + Sample E. The results are summarized in Figure 4.

### (3) Results and Consideration:

As can be seen from Figure 4, which shows the results of tracing the hybridization reactions for a period of 12000 seconds, the rate of hybrid-formation with ON1 + ON2, a fully-matched oligonucleotide pair, in which the base sequences of the oligonucleotides were completely complementary to each other, was much higher than those with ON1 + ON4, ON1 + ON5, and ON1 + ON6 which were mismatched oligonucleotide pairs. Specifically, after the progress of the hybridization reactions for 12000 seconds, a large number of hybrids were formed with ON1 + ON2, a fully-matched pair, whereas no sufficient amount of hybrids were formed with the mismatched pairs, in each of which there was one mismatch per 20 nucleotides.

Thus, the fully-matched oligonucleotide pair was markedly different from the mismatched pairs, in the time-course change in light absorbance, thereby enabling detection of the presence of the mismatch. It was noted, however, that, for the period of the hybridization reaction up to 12000 seconds, the time-course changes in absorbance among the mismatched pairs of ON1 + ON4, ON1 + ON5 and ON1 + ON6 were not so different as to make it possible to detect even the locations of the mismatches.

### Example 3: Hybridization by direct contact of oligonucleotides

This illustrates an example of the detection of an abnormal gene through a hybridization reaction in a reversed micelle into which there are injected a small amount of a first oligonucleotide and also a small amount of a second oligonucleotide having a completely complementary sequence or a mismatched sequence to the first oligonucleotide so that the two oligonucleotides are directly contacted with each other.

### (1) Formation of the reversed micelle:

To 1 ml of 50 mM AOT solution in iso-octance (an organic phase) was injected 9 µl of an aqueous solution of 198.0 µM Nucleotide 1 together with 10 mM Tris and 1 mM EDTA (2Na) having a pH of 7.0 to form a reversed micelle having a Wo of 10. Into the reversed micelle was injected 9 µl of an aqueous solution of 198.0 µM Nucleotide 2 together with 10mM Tris and 1 mM EDTA (2Na) having a pH of 7.0 to prepare a reversed micelle having a Wo of 20 (as Sample A). The sample was subjected to measurement at 15°C as shown below immediately after the preparation. In a similar manner there were prepared Samples B through E as summarized in Table 2, which were subjected to the measurements as shown below.

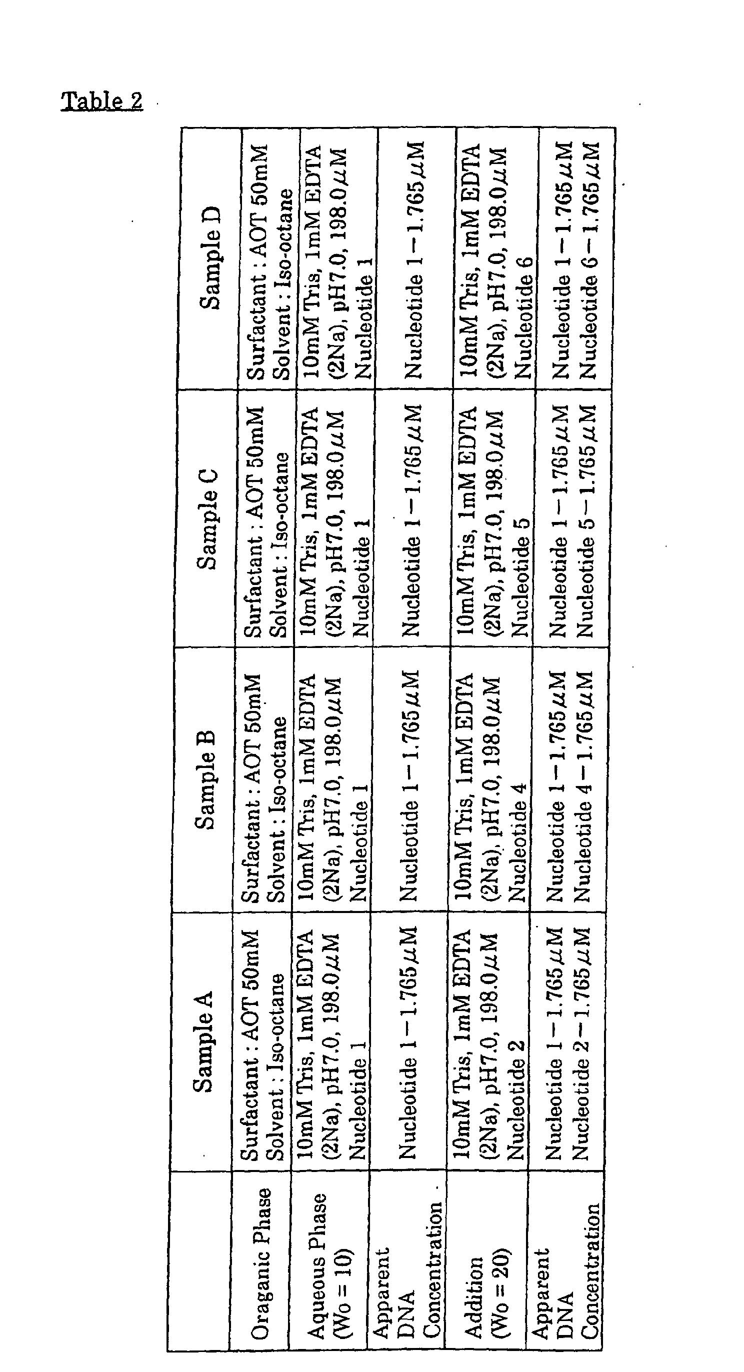

### (2) Measurements:

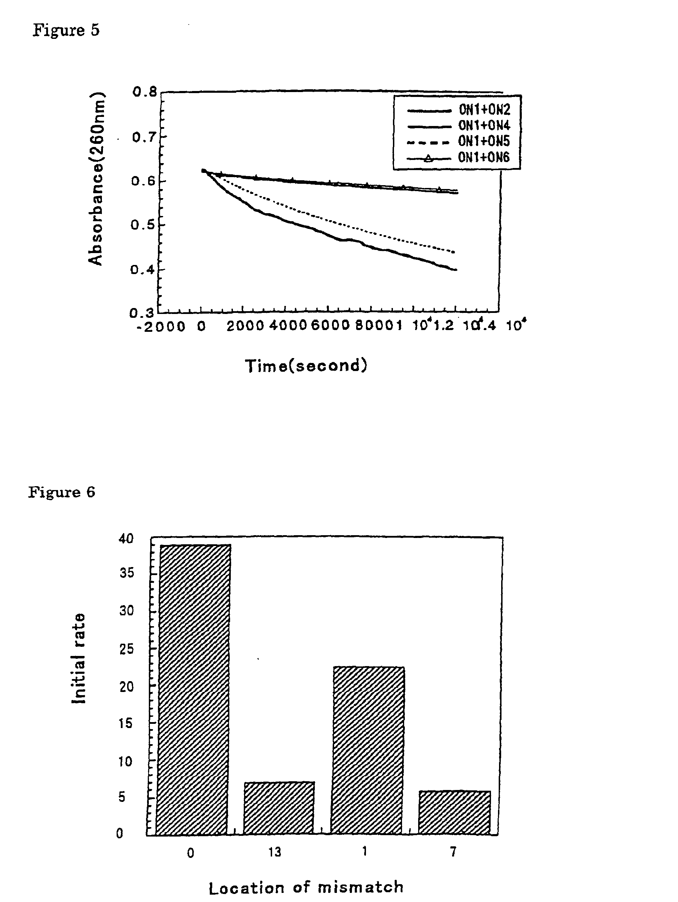

Each of Sample A, Sample B, Sample C and Sample D was measured for change in absorbance at 260 nm over the course of time as the hybridization reaction proceeded at 15°C. The results are shown in Figure 5. In addition, for each case the relative initial rate was calculated from the slope of the plot of the absorbance against the time of the hybridization reaction. The results are shown in Figure 6.

### (3) Results and Consideration:

As can be understood from the results shown in Figures 5 and 6, the method of this Example greatly enhances the rate of the reaction, as compared with that of Example 2, with an increased sensitivity for the detection of the presence of the mismatches, thus enabling even the detection of the locations of the mismatches. More specifically, it can be seen that the initial rate of the hybridization with the fully-matched pair is much higher than those with the mismatched pairs. Difference can be seen even among the mismatched pairs. The rate of the hybridization with the mismatched pair in which the mismatched site is located at the end of the sequence (the position 1: ON1 + ON5) is higher than those with the mismatched pairs in which the mismatched site is located at around the center of the sequence (the position 13: ON1 + ON4, the position 7: ON1 + ON6). Thus, the rate of hybridization reaction varies depending upon the location(s) of mismatch(es). Utilizing this fact it is possible to detect the location(s) of mismatched site(s) in an abnormal gene.

### Industrial Applicability

As obvious from the foregoing description, according to the present invention it is possible to detect not only the presence or absence of altered sites in an abnormal gene but also the locations of such sites, by an extremely simple method which comprises preparing a reversed micelle containing a target gene and a probe therefor and measuring the progress of the hybridization reaction in the reversed micelle by such means as an ultraviolet apparatus. Thus, the present invention provides a method for the detection of an abnormal gene that is markedly superior to conventional methods for gene detection in safety, ease of operation, detection sensitivity, cost and other aspects.

## Claims

1. A method for detecting an abnormal gene, which comprises hybridizing, in a reversed micelle, a nucleic acid oligomer having a base sequence of a normal gene of interest with a probe having a base sequence complementary to said base sequence of the normal gene; hybridizing, in another reversed micelle, the nucleic acid oligomer having the base sequence of the normal gene with a probe having a base sequence complementary to a base sequence of an abnormal gene partly altered from the base sequence of the normal gene; and measuring the rates of the hybridization reactions to examine the difference therebetween.

2. The method of claim 1, wherein the hybridization reactions are carried out, in a single reversed micelle into which there have been injected the nucleic acid oligomer having the base sequence of the normal gene and the probe having the base sequence complementary to the base sequence of the normal gene, and in another single reversed micelle into which there have been injected the nucleic acid oligomer having the base sequence of the normal gene and the probe having the base sequence complementary to the base sequence of the abnormal gene.

3. The method of claim 1 or 2, wherein the measurement of the rates of the hybridization reactions is carried out by measuring change in ultraviolet light absorbance.

4. The method of claim 3, wherein the absorbance at 260nm is measured.


SEQUENCE LISTING

<110> Kyushu TLO Company, Limited

<120> Method for detecting abnormal genes

<130> P0405T

<160> 6

<210> 1

<211> 20

<212> DNA

<213> Homo sapiens

<400> 1

gctttgaggt gcgtgtttgt

<210> 2

<211> 20

<212> DNA

<213> Homo sapiens

<400> 2

cgaaactcca cgcacaaaca

<210> 3

<211> 20

<212> DNA

<213> Artificial sequence

<400> 3

cgaaactcca cgaacaaaca

<210> 4
<211> 20
<212> DNA
<213> Artificial sequence
<400> 4
cgaaactcca cgtacaaaca

<210> 5
<211> 20
<212> DNA
<213> Artificial sequence
<400> 5
tgaaactcca cgcacaaaca

<210> 6
<211> 20
<212> DNA
<213> Artificial sequence
<400> 6
cgaaacccca cgcacaaaca